# EUROPEAN PATENT SPECIFICATION

(11) **EP 3 801 532 B1**
(45) Date of publication and mention of the grant of the patent: **05.11.2025**
(21) Application number: 19814132.7
(22) Date of filing: 31.05.2019
(51) Int. Cl.: A61K 31/513, A61K 9/20

(54) **FORMULATIONS OF RALTEGRAVIR**
FORMULIERUNGEN VON RALTEGRAVIR
PRÉPARATIONS DE RALTÉGRAVIR

(30) Priority: 06.06.2018 US 201862681163 P
(43) Date of publication of application: 14.04.2021
(73) Proprietor: Merck Sharp & Dohme LLC, Rahway, New Jersey 07065 (US)
(72) Inventor: MAHJOUR, Majid, West Point, Pennsylvania 19486 (US); SMITH, Ronald, L., Rahway, New Jersey 07065-0907 (US)
(74) Representative: Merck Sharp & Dohme LLC
(86) International application number: PCT/US2019/034782
(87) International publication number: WO 2019/236395

(56) References cited:
- WO-A1-2011/053504
- WO-A2-2006/060711
- US-A1- 2006 122 205
- US-A1- 2006 122 205
- US-A1- 2017 231 993
- US-A1- 2017 231 993

## Description

### FIELD OF THE INVENTION

The present invention is directed to compressed tablet formulations that contain raltegravir or a pharmaceutically acceptable salt thereof, and to methods of treating HIV, using said compressed tablet formulations.

### BACKGROUND OF THE INVENTION

Human immunodeficiency virus ("HIV") is a lentivirus (a member of the retrovirus family) that causes *acquired immunodeficiency syndrome* ("AIDS") (Weiss, R.A. (May 1993). "How does HIV cause AIDS?" Science 260 (5112): 1273-9. "Emerging concepts in the immunopathogenesis of AIDS". Annu. Rev. Med. 60: 471-84) a condition in humans in which the immune system begins to fail, leading to life-threatening opportunistic infections. Infection with HIV occurs by the transfer of blood, semen, vaginal fluid, pre-ejaculate, or breast milk. Within these bodily fluids, HIV is present as both free virus particles and virus within infected immune cells. Screening of blood products for HIV has largely eliminated transmission through blood transfusions or infected blood products in the developed world. HIV infection in humans is considered pandemic by the World Health Organization (WHO). From its discovery in 1981 to 2006, AIDS killed more than 25 million people. HIV infects about 0.6% of the world's population (Joint United Nations Programme on HIV/AIDS (2006). "Overview of the global AIDS epidemic" (PDF). 2006 Report on the global AIDS epidemic. ISBN 9291734799.).

The development of highly active antiretroviral therapy (HAART) in the mid 1990's transformed the clinical care of human immunodeficiency virus (HIV) type infection. HAART regimens have proven to be highly effective treatments, significantly decreasing HIV viral load in HIV-infected patients, thereby slowing the evolution of the illness and reducing HIV-related morbidity and mortality. Yet, the treatment success of HAART is directly related to adherence to the regimen by the patient. Unless appropriate levels of the antiretroviral drug combinations are maintained in the blood, viral mutations will develop, leading to therapy resistance and cross-resistances to molecules of the same therapeutic class, thus placing the long-term efficacy of treatments at risk. Various clinical studies have shown a decline in treatment effectiveness with relatively small lapses in adherence. A study by Musiime found that 81% of patients with more than 95% adherence demonstrated viral suppression, while only 50% of patients who were 80-90% adherent were successful. See, Musiime, S., et al., Adherence to Highly Active Antiretroviral Treatment in HIV-Infected Rwandan Women. PLOS one 2011, 6, (11), 1-6. Remarkably, only 6% of patients that were less than 70% adherent showed improvements in viral markers. Thus, low adherence is a leading cause of therapeutic failure in treatment of HIV-1 infection.

Nonetheless, adherence rates to the HAART regimens continue to be far from optimal. Various characteristics of HAART make adherence particularly difficult. Therapeutic regimens are complex, requiring multiple drugs to be taken daily, often at different times of the day, and many with strict requirements on food intake. Many HAART medications also have unpleasant side effects, including nausea, diarrhea, headache, and peripheral neuropathy. Social and psychological factors can also negatively impact adherence. Patients report that forgetfulness, lifestyle factors, including fear of being identified as HIV-positive, and therapy fatigue over life-long duration of treatment all contribute to adherence lapses. New HIV treatment interventions aim to improve adherence by reducing the complexity of treatments, the frequency of the dosages, and/or the side effects of the medications.

Raltegravir inhibits the catalytic activity of HIV integrase, an HIV encoded enzyme that is required for viral replication. Inhibition of integrase prevents the covalent insertion, or integration, of unintegrated linear HIV DNA into the host cell genome preventing the formation of the HIV provirus. The provirus is required to direct the production of progeny virus, so inhibiting integration prevents propagation of the viral infection. Thus, raltegravir is known to be useful in treating HIV, and the use of raltegravir in such treatment has received health authority approval in various countries. In the U.S., the FDA has approved the potassium salt of raltegravir as ISENTRESS^{®}. Adult treatment-naive patients, or adult patients who are virologically suppressed on an initial regimen of ISENTRESS^{®} 400 mg twice daily, can be administered 1200 mg (2 X 600 mg) film-coated ISENTRESS HD^{®} tablets orally, once daily, or one 400 mg film-coated ISENTRESS^{®} tablet orally, twice daily.

The 1200 mg daily dose of ISENTRESS HD^{®} is administered as two 600 mg tablets. Additional formulation approaches capable of delivering 1200 mg of raltegravir in a single tablet in an acceptable image size are desirable.

### SUMMARY OF THE INVENTION

The present invention is directed to compressed tablets for oral administration, which comprise:
(A) an intragranular component comprising:
   (i) the potassium salt of raltegravir, which is present on a free phenol basis in an amount of at least about 70 wt.%;
   (ii) an inert superdisintegrant, which is present in an amount in a range of from 5 wt. % to about 10 wt.%; and
   (iii) a binder, which is present in an amount in a range of from about 0.5 wt.% to about 5 wt.%;
   and
(B) an extragranular component comprising:
   (i) a superdisintegrant, which is present in an amount in a range of from about 7 wt.% to about 11 wt.%; and
   (ii) a lubricant, which is present in an amount in a range of from about 0.5 wt.% to about 2.5 wt.%;
   wherein the weight percent of each ingredient in the compressed tablet is based on the total weight of the compressed tablet and wherein the tablet is free of filler.

### DETAILED DESCRIPTION OF THE INVENTION

The present invention is directed to compressed tablets for oral administration, which comprise an intragranular component comprising an effective amount of the potassium salt of raltegravir, an inert superdisintegrant, and a binder; and an extragranular component comprising a superdisintegrant and a lubricant.

Raltegravir is known to be useful in treating HIV, and the use of raltegravir in such treatment has received health authority approval in various countries. In the U.S., the FDA has approved the potassium salt of raltegravir as ISENTRESS^{®} and ISENTRESS HD^{®}.

The chemical name for raltegravir potassium is *N*-[(4-Fluorophenyl)methyl]-1,6-dihydro5-hydroxy-1-methyl-2-[1-methyl-1-[[(5-methyl-1,3,4-oxadiazol-2-yl)carbonyl]amino]ethyl]-6-oxo-4pyrimidinecarboxamide monopotassium salt. The empirical formula is C₂0H₂₀FKN₆O₅ and the molecular weight is 482.51. Raltegravir potassium is a white to off-white powder. It is soluble in water, slightly soluble in methanol, very slightly soluble in ethanol and acetonitrile and insoluble in isopropanol. The chemical structure of raltegravir potassium is as follows:

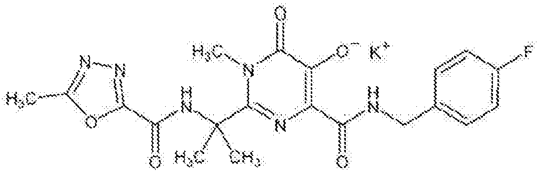

As used herein, "the potassium salt of raltegravir, which is present on a free phenol basis" describes the amount of raltegravir that is in free phenol form. For example, a 600 mg tablet of ISENTRESS^{®} contains 651.1 mg of raltegravir potassium, which is equivalent to 600 mg of raltegravir free phenol and a 1200 mg tablet of ISENTRESS^{®} contains 1303.20 mg of raltegravir potassium, which is equivalent to 1200 mg of raltegravir free phenol.

Various patents have been granted worldwide claiming raltegravir, related compounds and methods of using same. For example, U.S. Patent No. 7,169,780 is directed to a genus of hydroxypyrimidinone carboxamides, which includes raltegravir. U.S. Patent Nos. 7,217,713 and 7,435,734 are directed to methods of treating HIV and inhibiting HIV integrase, respectively. U.S. Patent No. 7,754,731 is directed to the anhydrous crystalline potassium salt of raltegravir. The international patent WO-2011053504 discloses a compressed tablet for oral administration, which comprises: (A) an intragranular component comprising:(i) an effective amount of an alkali metal salt of raltegravir, (ii) a first superdisintegrant, and (iii) a binder; and (B) an extragranular component comprising: (i) a second superdisintegrant, (ii) a filler, and (iii) a lubricant. These compressed tablets can suitably contain from about 50 mg to about 800 mg of alkali metal salt of raltegravir (e.g., potassium salt of raltegravir) per tablet.

The present invention is directed to compressed tablets for oral administration, which comprise:
(A) an intragranular component comprising:
   (i) the potassium salt of raltegravir, which is present on a free phenol basis in an amount of at least about 70 wt.%;
   (ii) an inert superdisintegrant, which is present in an amount in a range of from 5 wt. % to about 10 wt.%; and
   (iii) a binder, which is present in an amount in a range of from about 0.5 wt.% to about 5 wt.%;
   and
(B) an extragranular component comprising:
   (i) a superdisintegrant, which is present in an amount in a range of from about 7 wt.% to about **11** wt.%; and
   (ii) a lubricant, which is present in an amount in a range of from about 0.5 wt.% to about 2.5 wt.%;
   wherein the weight percent of each ingredient in the compressed tablet is based on the total weight of the compressed tablet and wherein the tablet is free of filler.

The compressed tablets of the instant invention comprise a high drug load of raltegravir potassium in single tablet, in an acceptable image size. In an embodiment of the invention, the raltegravir potassium comprises 81.45% (75% based on the free phenol form) of the tablet. Loading a high percentage of drug into a tablet, and having the resulting tablet have acceptable physical properties, is very challenging.

In general, disintegrants and superdisintegrants absorb water and expand when wet, causing the tablet to break apart in the digestive tract, releasing the active ingredient(s) for absorption. Superdisintegrants have a greater effectiveness at low concentrations (when compared with disintegrants) in the tablet. In some instances, the disintegrant or superdisintegrant can react with the active ingredient.

It has been observed that certain disintegrants react with raltegravir potassium, resulting in disproportionation; a portion of the raltegravir potassium is converted to the parent acid. Disproportionation of raltegravir potassium is an undesirable side reaction. To reduce the amount of disproportionation, the tablets of the instant invention employ an inert superdisintegrant in the intragranular component of the tablet. It is observed that the use of an inert superdisintegrant greatly reduces disproportionation in the instant tablets.

As used herein, the term "inert superdisintegrant" refers to a superdisintegrant that has a neutral pH and/or no functionality at the active site to react with other excipients or the active ingredient. In an embodiment of the invention, the inert superdisintegrant is selected from the group consisting of crospovidone, neutralized sodium croscarmellose and neutralized sodium starch glycolate. The neutralized sodium croscarmellose and neutralized sodium starch glycolate are neutralized with a base to pH ~7. In a class of the invention, the inert superdisintegrant is crospovidone.

The tablets of the instant invention have a small percentage of binder, and are free of filler. In general, the presence of fillers aids in tablet compressibility and enhances the physical properties of tablets. Surprisingly, the tablets of the instant invention, which do not contain filler, are both robust and compressible, resulting in tablets with acceptable physical properties.

In general, binders hold the ingredients in a tablet together. In an embodiment of the invention, the binder is selected from the group consisting of hypromellose and hydroxypropyl cellulose. In a class of the invention, the binder is hypromellose.

In an embodiment of the invention, the superdisintegrant is selected from the group consisting of sodium croscarmellose, starch and sodium starch glycolate. In a class of the invention, the superdisintegrant is sodium croscarmellose.

In general, lubricants prevent ingredients from clumping together and from sticking to the tablet punches. In an embodiment of the invention, the lubricant is selected from the group consisting of magnesium stearate, stearic acid, sodium stearyl fumarate and mixtures thereof. In a class of the invention, the lubricant is magnesium stearate.

Excipients that are mixed with other ingredients (including raltegravir potassium) prior to granulation, and are thus incorporated within the granules, comprise the intragranular component of the formulation. Excipients that are mixed with the dry granules before the complete mixture is compacted comprise the extrangraular component of the formulation.

In an embodiment of the invention, the compressed tablet for oral administration comprises:
(A)(i) the potassium salt of raltegravir is present on a free phenol basis in an amount of at least about 75 wt.%;
(A)(ii) the inert superdisintegrant is present in an amount in a range of from 7 wt. % to about 9 wt.%;
(A)(iii) the binder is present in an amount in a range of from about 1.0 wt.% to about 3.0 wt.%;
(B)(i) the superdisintegrant is present in an amount in a range of from about 7 wt.% to about 11 wt.%; and
(B)(ii) the lubricant is present in an amount in a range of from about 1.0 wt.% to about 2.0 wt.%;
wherein the weight percent of each ingredient in the compressed tablet is based on the total weight of the compressed tablet and wherein the tablet is free of filler.

In an embodiment of the invention, the potassium salt of raltegravir is present on a free phenol basis is in an amount of 1200 mg per unit dose. In another embodiment of the invention, the potassium salt of raltegravir is present on a free phenol basis is in an amount of 600 mg per unit dose. In another embodiment of the invention, the potassium salt of raltegravir is present on a free phenol basis is in an amount of 300 mg per unit dose.

Exemplifying the instant invention is the following compressed tablet:

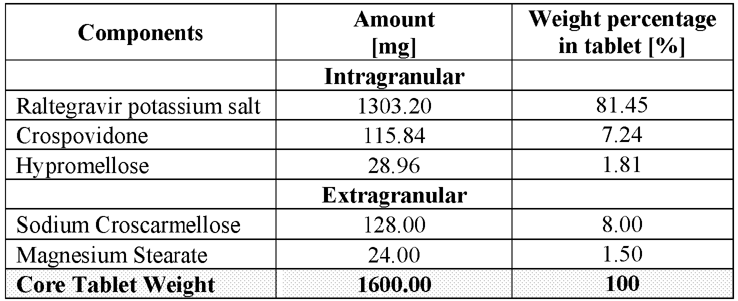

Also exemplifying the instant invention is the following compressed tablet:

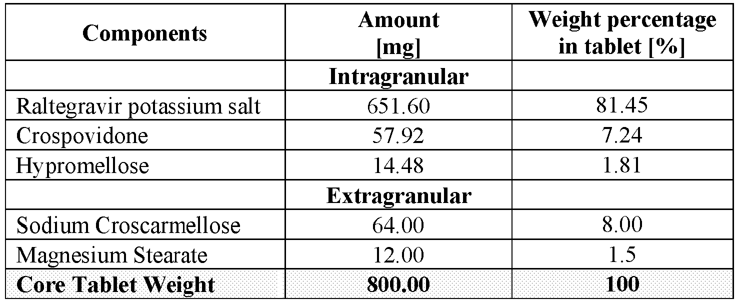

Also exemplifying the instant invention is the following compressed tablet:

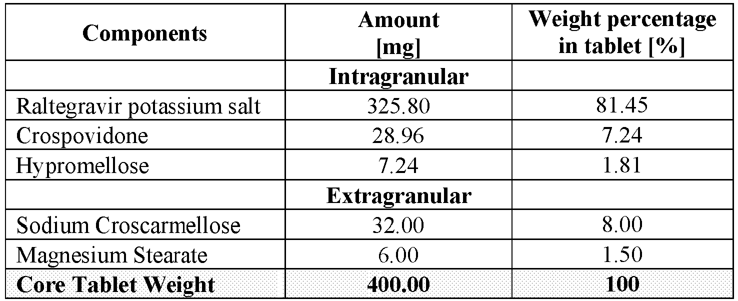

The pharmaceutical tablet compositions of the present invention may also contain one or more additional formulation ingredients that may be selected from a wide variety of excipients known in the pharmaceutical formulation art. According to the desired properties of the tablet, any number of ingredients may be selected, alone or in combination, based upon their known uses in preparing tablet compositions. Such ingredients include, but are not limited to, flavors, flavor enhancers, sweeteners, preservatives, colorants and coatings.

The term "tablet" or "compressed tablet" as used herein is intended to encompass compressed pharmaceutical dosage formulations of all shapes and sizes, whether uncoated or coated. Substances which may be used for coating include hydroxypropylmethylcellulose, hydroxypropylcellulose, titanium dioxide, talc, sweeteners and colorants.

### Treatment or Prevention of HIV Infection

Raltegravir is known to be useful in treating HIV infection. Raltegravir may be useful in treating or preventing infection by HIV after suspected past exposure to HIV by such means as blood transfusion, exchange of body fluids, bites, accidental needle stick, or exposure to subject blood during surgery or other medical procedures.

### Combination Therapy

In another embodiment of the instant invention, the compressed tablets of the instant invention can be combined with one or more additional therapeutic agents present methods useful for treating or preventing HIV infection.

In one embodiment, the additional therapeutic agent is an antiviral agent.

In another embodiment, the additional therapeutic agent is an immunomodulatory agent, such as an immunosuppressive agent.

Accordingly, in one embodiment, the present invention provides methods for treating a viral infection in a subject, the method comprising administering to the subject: (i) the compressed tablets of the invention, and (ii) at least one additional therapeutic agent that is other than raltegravir potassium, wherein the amounts administered are together effective to treat or prevent a viral infection.

When administering a combination therapy of the invention to a subject, therapeutic agents in the combination, or a pharmaceutical composition or compositions comprising therapeutic agents, may be administered in any order such as, for example, sequentially, concurrently, together, simultaneously and the like. The amounts of the various actives in such combination therapy may be different amounts (different dosage amounts) or same amounts (same dosage amounts). Thus, for non-limiting illustration purposes, compressed tablets of the instant invention and an additional therapeutic agent may be present in fixed amounts (dosage amounts) in a single dosage unit (*e.g.,* a capsule, a tablet and the like).

In one embodiment, at least one compressed tablet of the instant invention is administered during a time when the additional therapeutic agent(s) exert their prophylactic or therapeutic effect, or *vice versa.*

In another embodiment, at least one compressed tablet of the instant invention is administered in doses commonly employed when such agents are used as monotherapy for treating a viral infection.

In another embodiment, at least one compressed tablet of the instant invention and the additional therapeutic agent(s) are administered in doses lower than the doses commonly employed when such agents are used as monotherapy for treating a viral infection.

In still another embodiment, at least one compressed tablet of the instant invention and the additional therapeutic agent(s) act synergistically and are administered in doses lower than the doses commonly employed when such agents are used as monotherapy for treating a viral infection.

Viral infections and virus-related disorders that may be treated or prevented using the combination therapy methods of the present invention include, but are not limited to, those listed above.

In one embodiment, the viral infection is HIV infection.

In another embodiment, the viral infection is AIDS.

The compressed tablets of the instant invention and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

In one embodiment, the administration of a compressed tablet of the instant invention and the additional therapeutic agent(s) may inhibit the resistance of a viral infection to these agents.

As noted above, the present invention is also directed to use of a compressed tablet of the instant invention with one or more anti-HIV agents. An "anti-HIV agent" is any agent which is directly or indirectly effective in the inhibition of HIV reverse transcriptase or another enzyme required for HIV replication or infection, the treatment or prophylaxis of HIV infection, and/or the treatment, prophylaxis or delay in the onset or progression of AIDS. It is understood that an anti-HIV agent is effective in treating, preventing, or delaying the onset or progression of HIV infection or AIDS and/or diseases or conditions arising therefrom or associated therewith. For example, compressed tablets of the instant invention may be effectively administered, whether at periods of pre-exposure and/or post-exposure, in combination with effective amounts of one or more anti-HIV agents selected from HIV antiviral agents, immunomodulators, antiinfectives, or vaccines useful for treating HIV infection or AIDS. Suitable HIV antivirals for use in combination with the compressed tablets of the instant invention include, for example, those listed in Table A as follows:

**Table A**

| **Name** | **Trade Name** |
|---|---|
| abacavir, ABC | Ziagen^{®} |
| abacavir +lamivudine | Epzicom^{®} |
| abacavir + lamivudine + zidovudine | Trizivir^{®} |
| amprenavir | Agenerase^{®} |
| atazanavir | Reyataz^{®} |
| AZT, zidovudine, azidothymidine | Retrovir^{®} |
| darunavir | Prezista^{®} |
| ddC, zalcitabine, dideoxycytidine | Hivid^{®} |
| ddI, didanosine, dideoxyinosine | Videx^{®} |
| ddI (enteric coated) | Videx EC^{®} |
| delavirdine, DLV | Rescriptor^{®} |
| dolutegravir | Tivicay^{®} |
| doravirine | Pifeltro^{®} |
| doravirine + lamivudine + tenofovir DF | Delstrigo^{®} |
| efavirenz, EFV | Sustiva^{®}, Stocrin^{®} |
| efavirenz + emtricitabine + tenofovir DF | Atripla^{®} |
| EFdA (4'-ethynyl-2-fluoro-2'-deoxyadenosine) | |
| emtricitabine, FTC | Emtriva^{®} |
| emtricitabine + tenofovir DF | Truvada^{®} |
| emvirine | Coactinon^{®} |
| enfuvirtide | Fuzeon^{®} |
| enteric coated didanosine | Videx EC^{®} |
| etravirine, TMC-125 | Intelence^{®} |
| fosamprenavir calcium | Lexiva^{®} |
| indinavir | Crixivan^{®} |
| lamivudine, 3TC | Epivir^{®} |
| lamivudine + zidovudine | Combivir^{®} |
| lopinavir | |
| lopinavir + ritonavir | Kaletra^{®} |
| maraviroc | Selzentry^{®} |
| nelfinavir | Viracept^{®} |
| nevirapine, NVP | Viramune^{®} |
| rilpivirine, TMC-278 | Edurant^{®} |
| ritonavir | Norvir^{®} |
| saquinavir | Invirase^{®}, Fortovase^{®} |
| stavudine, d4T,didehydrodeoxythymidine | Zerit^{®} |
| tenofovir DF (DF = disoproxil fumarate), TDF | Viread^{®} |
| tipranavir | Aptivus^{®} |

Some of the drugs listed in the table are used in a salt form; e.g., abacavir sulfate, indinavir sulfate, atazanavir sulfate, nelfinavir mesylate.

In one embodiment, one or more anti-HIV drugs are selected from, lamivudine, abacavir, ritonavir, darunavir, atazanavir, emtricitabine, tenofovir, rilpivirine, doravirine, EFdA and lopinavir.

In another embodiment, the compressed tablets of the instant invention are used in combination with lamivudine.

In still another embodiment, the compressed tablets of the instant invention are used in combination with atazanavir.

In another embodiment, the compressed tablets of the instant invention are used in combination with darunavir.

In another embodiment the compressed tablets of the instant invention are used in combination with rilpivirine.

In one embodiment, the compressed tablets of the instant invention are used in combination with lamivudine and abacavir.

In another embodiment, the compressed tablets of the instant invention are used in combination with doravirine.

In another embodiment, the compressed tablets of the instant invention are used in combination with emtricitabine and tenofovir.

In another embodiment, the compressed tablets of the instant invention are used in combination with EFdA.

In another embodiment, the compressed tablets of the instant invention are used in combination with lopinavir and ritonavir.

In another embodiment, the present invention provides a method for the treatment or prophylaxis of infection by HIV or for the treatment, prophylaxis, or delay in the onset or progression of AIDS in a subject in need thereof, which comprises administering to the subject (i) the compressed tablets of the instant invention are and (ii) one or more additional anti-HIV agents selected from lamivudine, abacavir, ritonavir and lopinavir, or a pharmaceutically acceptable salt or prodrug thereof, wherein the amounts administered of components (i) and (ii) are together effective for the treatment or prophylaxis of infection by HIV or for the treatment, prophylaxis, or delay in the onset or progression of AIDS in the subject in need thereof.

It is understood that the scope of combinations of the compounds of this invention with anti-HIV agents is not limited to the HIV antivirals listed in Table A, but includes in principle any combination with any pharmaceutical composition useful for the treatment or prophylaxis of AIDS. The HIV antiviral agents and other agents will typically be employed in these combinations in their conventional dosage ranges and regimens as reported in the art, including, for example, the dosages described in the Physicians' Desk Reference, Thomson PDR, Thomson PDR, 65th edition (2011), the 66th edition (2012), at www.pdr.net,and the like. The dosage ranges for a compound of the invention in these combinations are the same as those set forth above.

The doses and dosage regimen of the other agents used in the combination therapies of the present invention for the treatment or prevention of HIV infection may be determined by the attending clinician, taking into consideration the approved doses and dosage regimen in the package insert; the age, sex and general health of the subject; and the type and severity of the viral infection or related disease or disorder. When administered in combination, the compressed tablets of the instant invention and the other agent(s) may be administered simultaneously (*i.e.,* in the same composition or in separate compositions one right after the other) or sequentially. This is particularly useful when the components of the combination are given on different dosing schedules, e.g., one component is administered once daily and another component is administered every six hours, or when the pharmaceutical compositions are different, e.g., one is a tablet and one is a capsule. A kit comprising the separate dosage forms is therefore advantageous.

The following examples are given for the purpose of illustrating the present invention and shall not be construed as being limitations on the scope of the invention.

### EXAMPLE 1

### PREPARATION OF 300, 600 AND 1200MG COMPRESSED TABLETS

The following tablets were prepared from granulation containing 85.71% raltegravir potassium and intragranular and extragranular croscarmellose sodium:

| Component | Percentage in tablet% | mg/600mg tablet | mg/300g tablet | mg/1200mg tablet |
|---|---|---|---|---|
| Raltegravir K salt (salt factor = 1.086) | 62.06 | 651.60 | 325.80 | 1303.20 |
| Croscarmellose Sodium | 6.21 | 65.16 | 32.58 | 130.32 |
| HPMC (6cp) | 4.14 | 43.44 | 21.72 | 86.88 |
| weight of granulation/ intragranular (mg) | 72.40 | 760.20 | 380.10 | 1520.40 |
| microcrystalline cellulose (Avicel 102) | 17.10 | 179.55 | 89.78 | 359.10 |
| Croscarmellose Sodium | 9.00 | 94.50 | 47.25 | 189.00 |
| MgSt. | 1.50 | 15.75 | 7.88 | 31.50 |
| Total Image weight (mg) | 100.0 | 1050.0 | 525.0 | 2100.0 |

Compressed tablets containing 300 and 600 mg of raltegravir on a free phenol basis were prepared by first blending a mixture (about 4 kg) of the raltegravir K salt, the HPMC and the intragranular portion of croscarmellose sodium in a Fielder 10/25L high shear granulator at an impeller speed of 500 rpm and a chopper speed of 1800 rpm for 0.5 minute, then adding USP water (40 wt.% ; about 1.6 kg) to the granulator and granulating at 250 rpm for 5 minutes at a spray rate of 320 g/minute. The granulated material was then dried in a GPG-3 fluid bed granulator at an inlet air temperature of 80°C for 20-30 minutes to afford a dried granulate with a Loss on Drying ("LOD") of about 1 wt.%. The dried granulate was then milled and screened using a Quadro 197 Comil with a square bar operated at 2000 rpm fitted with a grated screen having a 1.27 mm opening (i.e., No. 50 screen) to provide the granules. The granules were then blended with the microcrystalline cellulose and the extragranular portion of croscarmellose sodium in a 8-quart V-shell blender at an rotation speed of 25 rpm for 5 minutes. Magnesium stearate (pre-screened using a No. 40 mesh size screen) was then added to the blender and the mixture was blended for 5 more minutes at an impeller speed of 25 rpm. The lubricated granules were then compressed into tablets using on an **RRDI** Compaction Simulator tablet press with image weights corresponding to 300 and 600 mg tablets. The 1200 mg tablets were not made; the image was too large for the tablet punches. While the 300 mg and 600 mg tablets are an acceptable image size, the 1200 mg tablets, which bear a total image weight of 2100 mg, are too large for human consumption.

### EXAMPLE 2

### PREPARATION OF 1200MG COMPRESSED TABLETS

The following tablets were prepared from granulation containing 85.71% raltegravir potassium and intragranular croscarmellose sodium and various extragranular excipient combinations:

| | | Formulation | | | | | |
|---|---|---|---|---|---|---|---|
| Ingredients | mg/1200mg tablet (from Example 1) | A | B | C | D | E | F |
| RaltegravirK salt (salt factor = 1.086) (intra) | 1303.2 | 1303.2 | 1303.2 | 1303.2 | 1303.2 | 1303.2 | 1303.2 |
| Croscarmellose Sodium (Intra) | 130.32 | 130.32 | 130.32 | 130.32 | 130.32 | 130.32 | 130.32 |
| HPMC (6cp) (intra) | 86.88 | 86.88 | 86.88 | 86.88 | 86.88 | 86.88 | 86.88 |
| Granules | 1520.4 | 1520.4 | 1520.4 | 1520.4 | 1520.4 | 1520.4 | 1520.4 |
| microcrystalline cellulose (Avicel 102) | 359.1 | | | | | | |
| Amberlite IR88 Polacrilin Potassium NF | | | 104.85 | | | | |
| Pharmaburst 500 | | | | 104.85 | | | |
| Tri-Calcium Phosphate | | 48 | | | 104.85 | | |
| Croscarmellose Sodium | 189 | 56.85 | | | | 104.85 | |
| Crospovidone | | | | | | | 104.85 |
| MgSt. | 31.5 | 24.75 | 24.75 | 24.75 | 24.75 | 24.75 | 24.75 |
| Image (mg) | 2100 | 1650 | 1650 | 1650 | 1650 | 1650 | 1650 |

Compressed tablets containing 1200 mg of raltegravir on a free phenol basis were prepared from a granulation containing 85.71% raltegravir potassium. The granulation was prepared by first blending a mixture (about 4 kg) of the raltegravir K salt, the HPMC and the intragranular portion of croscarmellose sodium in a Fielder 10/25L high shear granulator at an impeller speed of 500 rpm and a chopper speed of 1800 rpm for 0.5 minute, then adding USP water (40 wt.% ; about 1.6 kg) to the granulator and granulating at 250 rpm for 5 minutes at a spray rate of 320 g/minute. The granulated material was then dried in a GPG-3 fluid bed granulator at an inlet air temperature of 80°C for 20-30 minutes, wherein the air flow rate was gradually reduced during the drying period to afford a dried granulate with an LOD of about 1 wt.%. The dried granulate was then milled and screened using a Quadro 197 Comil with a square bar operated at 2000 rpm fitted with a grated screen having a 1.27 mm opening (i.e., No. 50 screen) to provide the granules. A 280g portion of the dried granules and the extragranular excipients were added to a 1.25 L bowl and blended on Bohl blender at a rotation speed of 25 rpm for 5 minutes, or in a bottle and blended on Turbula blender for 5 minutes. Magnesium stearate (pre-screened using a No. 40 mesh size screen) was then added to the blender and the granules were lubricated for 5 minutes at 25 rpm. The lubricated granules were then compressed into image weight for the 1200 mg dose (1650 mg image) tablets using a rotary tablet press with 0.850"X0.445" oval shaped tooling at a compression force necessary to achieve a tablet hardness of about ~30 kiloponds. Tablets A - F had disintegration times of greater than 30 minutes in 0.08 N HCl.

### EXAMPLE 3

### PREPARATION OF GRANULATIONS CONTAINING >85.7% RALTEGRAVIR POTASSIUM BY HIGH SHEAR WET GRANULATION AND FLUID BED DRYING

| | Granulation G | Granulation H | Granulation I | Granulation J |
|---|---|---|---|---|
| Ingredients | % | % | % | % |
| Raltegravir K salt (salt factor = 1.086) | 90.00 | 90.00 | 87.00 | 86.00 |
| Crospovidone | 8.00 | 8.00 | 12.00 | 12.00 |
| HPMC (6cp) | 2.00 | 2.00 | | 2.00 |
| Carbomer 934P [Carbopol 974P] | | | 1.00 | |
| GFL (water)* | 30.00 | 27.00 | 33.00 | 32.00 |
| % Granules | 100.00 | 100.00 | 100.00 | 100.00 |
| Weight of granules for 1200mg tablet (See Example 4) | 1448 | 1448 | 1483 | 1515 |
| * will be removed during process | | | | |

The granulation was prepared by first blending a mixture (about 280 g) of the raltegravir K salt, the HPMC and the intragranular portion of superdisintegrant in a Diosna 2L high shear granulator at an impeller speed of 1000 rpm and a chopper speed of 2000 rpm for 1 minute, then adding USP water (~27-33% wt.% ; about 90 g) to the granulator and granulating at 580 rpm for 5 minutes at a spray rate of ~18 g/minute. The granulated material was then dried in a MP1 Niro Aeromatic Fluid Bed Dyer at an inlet air temperature of 80°C for 15-20 minutes to afford a dried granulate with an LOD of about 1 wt.%. The dried granulate was then milled and screened using a Quadro 197 Comil with a square bar operated at 2000 rpm fitted with a grated screen having a 1.27 mm opening (i.e., No. 50 screen) to provide the milled granules. Granulations G, H, I and J were used to make the tablets in Example 4.

### EXAMPLE 4

### PREPARATION OF 1200MG COMPRESSED TABLETS

Tablets in Example 4 were prepared from granulation containing > 85.71% raltegravir potassium (from Example 3), intragranular crospovidone and various extragranular excipient combinations

| | Formulation | | | | | | |
|---|---|---|---|---|---|---|---|
| Ingredients | K (from I granules) | L (from J granules) | M (from G granules) | N (from G granules) | O (from G granules) | P (from H granules) | Q (from H granules) |
| Raltegravir K salt (salt factor = 1.086) | 1303.20 | 1303.20 | 1303.02 | 1303.20 | 1303.20 | 1303.20 | 1303.20 |
| Crospovidone | 179.75 | 181.84 | 115.84 | 115.84 | 115.84 | 115.84 | 115.84 |
| HPMC (6cp) | 0.00 | 30.31 | 28.96 | 28.96 | 28.96 | 28.96 | 28.96 |
| Carbomer 934P [Carbopol 974P] | 14.98 | | | | | | |
| Granules | 1497.93 | 1515.35 | 1448.00 | 1448.00 | 1448.00 | 1448.00 | 1448.00 |
| Crospovidone | 51.55 | 48.85 | 128.00 | - | | | |
| Tri-Calcium Phosphate | | | | | 16.00 | 16.00 | |
| Sodium Croscarmellose | | | | 128.00 | 128.00 | 128.00 | 128.00 |
| MgSt | 23.60 | 23.80 | 24.00 | 24.00 | 24.00 | 24.00 | 24.00 |
| Total | 1573.08 | 1588.00 | 1600.00 | 1600.00 | 1616.00 | 1616.00 | 1600.00 |
| Disintegration time (m:s) in 0.08 N HCl | ~30:00 | ~27:30 | 20:41 | 18:49 | 16:16 | 18:19 | 15:26 |

The milled granules from Example 3 were blended with the extragranular excipients in a 1.25 L bowl and blended on a Bohl blender at a rotation speed of 25 rpm for 5 minutes, or in bottles and blended on Turbula blender for 5 minutes. Magnesium stearate (pre-screened using a No. 40 mesh size screen) was then added to the blender and the mixture blended for 5 minutes at 25 rpm. The lubricated granules were then compressed into 1573-1616 mg image weight for the1200 mg dose tablets using an RRDI compaction simulator tablet press with 0.840"x0.445" oval shaped tooling at a compression force necessary to achieve a tablet hardness of about ~30 kiloponds. All the blends made from above granulations (from Example 3) and extragranular excipients had good tensile strength (>2MPa tensile strength at 200 MPa compressive stress). The tablet formulations containing granulation G and sodium croscarmellose or sodium croscarmellose/calcium phosphate had ≤ 20 min disintegration times in 0.08 N HCl.

### EXAMPLE 5

### 13C _{SS}NMR STUDIES

To measure and compare the amount of raltegravir potassium disproportionation to its free phenol during granulation, wherein the granules contained either crospovidone or sodium croscarmellose as a superdisintegrant in the intrangraular component of the formulation, the granules were analyzed using ¹³C ssNMR as described below:
The ¹³C ssNMR spectra were acquired on a Bruker Avance spectrometer (Billerica, MA) operating at proton frequency of 400.13 MHz. Granules were packed into a 4 mm outer diameter zirconia rotor. The rotor was then inserted into a triple resonance (HXY) solid-state NMR probe head. Data were collected using a cross polarization magic angle spinning (CPMAS) pulse sequence with SPINAL-64 proton decoupling. ¹³C spectra were recorded at 298 K temperature at the spinning speed of 10 kHz. Spectra of a known mass of neat K salt and neat free phenol were also acquired as reference standards.

Based on previous work, peaks near 152 and 145.5 ppm were used to quantify extent of disproportionation. Spectra were deconvoluted using TopSpin software. The line shapes of the reference samples were held constant for the analyte samples. Only the intensity and chemical shift (to allow for field drift between experiments) were allowed to vary during the fitting. The resulting intensities were used to predict masses of each component within the analyte samples.

The granulation with crospovidone as the intragranular disintegrant showed about 0.4% disproportionation, where as the granulation with sodium croscarmellose had about 2.2% free phenol. Thus, the use of the inert superdisintegrant crospovidone instead of sodium croscarmellose reduced the extent of disproportionation by about 5 times.

## Claims

1. A compressed tablet for oral administration, which comprises :
(A) an intragranular component comprising:
(i) the potassium salt of raltegravir, which is present on a free phenol basis in an amount of at least about 70 wt.%;
(ii) an inert superdisintegrant, which is present in an amount in a range of from 5 wt. % to about 10 wt.%; and
(iii) a binder, which is present in an amount in a range of from about 0.5 wt.% to about 5 wt.%;
and
(B) an extragranular component comprising:
(i) a superdisintegrant, which is present in an amount in a range of from about 7 wt.% to about 11 wt.%; and
(ii) a lubricant, which is present in an amount in a range of from about 0.5 wt.% to about 2.5 wt.%;
wherein the weight percent of each ingredient in the compressed tablet is based on the total weight of the compressed tablet and wherein the tablet is free of filler.

2. The compressed tablet of Claim 1 wherein the inert superdisintegrant is selected from the group consisting of crospovidone, neutralized sodium croscarmellose and neutralized sodium starch glycolate.

3. The compressed tablet of Claim 2 wherein the inert superdisintegrant is crospovidone.

4. The compressed tablet of Claim 1 wherein the binder is selected from the group consisting of hypromellose and hydroxypropyl cellulose.

5. The compressed tablet of Claim 4 wherein the binder is hypromellose.

6. The compressed tablet of Claim 1 wherein the superdisintegrant is selected from the group consisting of sodium croscarmellose, starch and sodium starch glycolate.

7. The compressed tablet of Claim 6 wherein the superdisintegrant is sodium croscarmellose.

8. The compressed tablet of Claim 1 wherein the lubricant is selected from the group consisting of magnesium stearate, stearic acid, sodium stearyl fumarate and mixtures thereof.

9. The compressed tablet of Claim 8 wherein the lubricant is magnesium stearate.

10. The compressed tablet of Claim 1 wherein:
(A)(i) the potassium salt of raltegravir is present on a free phenol basis in an amount of at least about 75 wt.%;
(A)(ii) the inert superdisintegrant is present in an amount in a range of from 7 wt. % to about 9 wt.%;
(A)(iii) the binder is present in an amount in a range of from about 1.0 wt.% to about 3.0 wt.%;
(B)(i) the superdisintegrant is present in an amount in a range of from about 7 wt.% to about 11 wt.%; and
(B)(ii) the lubricant is present in an amount in a range of from about 1.0 wt.% to about 2.0 wt.%;
wherein the weight percent of each ingredient in the compressed tablet is based on the total weight of the compressed tablet.

11. The compressed tablet of Claim 1 wherein the potassium salt of raltegravir is present on a free phenol basis is in an amount of 1200 mg per unit dose.

12. The compressed tablet of Claim 1 wherein the potassium salt of raltegravir is present on a free phenol basis is in an amount of 600 mg per unit dose.

13. The compressed tablet of Claim 1 wherein the potassium salt of raltegravir is present on a free phenol basis is in an amount of 300 mg per unit dose.

14. The compressed tablet of Claim 1 comprising:
| **Components** | **Amount [mg]** |
|---|---|
| | **Intragranular** |
| Raltegravir potassium salt | 1303.20 |
| Crospovidone | 115.84 |
| Hypromellose | 28.96 |
| | **Extragranular** |
|---|---|
| Sodium Croscarmellose | 128.00 |
| Magnesium Stearate | 24.00 |
| **Core Tablet Weight** | **1600.00** |

15. The compressed tablet of Claim 1 comprising:
| **Components** | **Amount [mg]** |
|---|---|
| | **Intragranular** |
| Raltegravir potassium salt | 651.60 |
| Crospovidone | 57.92 |
| Hypromellose | 14.48 |
| | **Extragranular** |
|---|---|
| Sodium Croscarmellose | 64.00 |
| Magnesium Stearate | 12.00 |
| **Core Tablet Weight** | **800.00** |

16. The compressed tablet of Claim 1 comprising:
| **Components** | **Amount [mg]** |
|---|---|
| | **Intragranular** |
| Raltegravir potassium salt | 325.80 |
| Crospovidone | 28.96 |
| Hypromellose | 7.24 |
| | **Extragranular** |
|---|---|
| Sodium Croscarmellose | 32.00 |
| Magnesium Stearate | 6.00 |
| **Core Tablet Weight** | **400.00** |

## Patentansprüche

1. Gepresste Tablette zur oralen Verabreichung, umfassend:
(A) eine intragranulare Komponente, umfassend:
(i) das Kaliumsalz von Raltegravir, das in einer Menge von wenigstens etwa 70 Gew.-%, auf Basis von freiem Phenol, vorliegt,
(ii) ein inertes Supersprengmittel, das in einer Menge in einem Bereich von 5 Gew.-% bis etwa 10 Gew.-% vorliegt, und
(iii) ein Bindemittel, das in einer Menge in einem Bereich von etwa 0,5 Gew.-% bis etwa 5 Gew.-% vorliegt,
und
(B) eine extragranulare Komponente, umfassend:
(i) ein Supersprengmittel, das in einer Menge in einem Bereich von etwa 7 Gew.-% bis etwa 11 Gew.-% vorliegt, und
(ii) ein Gleitmittel, das in einer Menge in einem Bereich von etwa 0,5 Gew.-% bis etwa 2,5 Gew.-% vorliegt,
wobei die Gewichtsprozente eines jeden Inhaltsstoffs in der gepressten Tablette auf das Gesamtgewicht der gepressten Tablette bezogen sind, und wobei die Tablette frei von Füllstoff ist.

2. Gepresste Tablette nach Anspruch 1, wobei das inerte Supersprengmittel ausgewählt ist aus der Gruppe bestehend aus Crospovidon, neutralisiertem Croscarmellose-Natrium und neutralisiertem Natriumstärkeglykolat.

3. Gepresste Tablette nach Anspruch 2, wobei das inerte Supersprengmittel Crospovidon ist.

4. Gepresste Tablette nach Anspruch 1, wobei das Bindemittel ausgewählt ist aus der Gruppe bestehend aus Hypromellose und Hydroxypropylcellulose.

5. Gepresste Tablette nach Anspruch 4, wobei das Bindemittel Hypromellose ist.

6. Gepresste Tablette nach Anspruch 1, wobei das Supersprengmittel ausgewählt ist aus der Gruppe bestehend aus Croscarmellose-Natrium, Stärke und Natriumstärkeglykolat.

7. Gepresste Tablette nach Anspruch 6, wobei das Supersprengmittel Croscarmellose-Natrium ist.

8. Gepresste Tablette nach Anspruch 1, wobei das Gleitmittel ausgewählt ist aus der Gruppe bestehend aus Magnesiumstearat, Stearinsäure, Natriumstearylfumarat und Mischungen davon.

9. Gepresste Tablette nach Anspruch 8, wobei das Gleitmittel Magnesiumstearat ist.

10. Gepresste Tablette nach Anspruch 1, wobei:
(A)(i) das Kaliumsalz von Raltegravir in einer Menge von wenigstens etwa 75 Gew.-%, auf Basis von freiem Phenol, vorliegt,
(A)(ii) das inerte Supersprengmittel in einer Menge in einem Bereich von 7 Gew.-% bis etwa 9 Gew.-% vorliegt,
(A)(iii) das Bindemittel in einer Menge in einem Bereich von etwa 1,0 Gew.-% bis etwa 3,0 Gew.-% vorliegt,
(B)(i) das Supersprengmittel in einer Menge in einem Bereich von etwa 7 Gew.-% bis etwa 11 Gew.-% vorliegt, und
(B)(ii) das Gleitmittel in einer Menge in einem Bereich von etwa 1,0 Gew.-% bis etwa 2,0 Gew.-% vorliegt,
wobei die Gewichtsprozente eines jeden Inhaltsstoffs in der gepressten Tablette auf das Gesamtgewicht der gepressten Tablette bezogen sind.

11. Gepresste Tablette nach Anspruch 1, wobei das Kaliumsalz von Raltegravir in einer Menge von 1200 mg pro Dosierungseinheit, auf Basis von freiem Phenol, vorliegt.

12. Gepresste Tablette nach Anspruch 1, wobei das Kaliumsalz von Raltegravir in einer Menge von 600 mg pro Dosierungseinheit, auf Basis von freiem Phenol, vorliegt.

13. Gepresste Tablette nach Anspruch 1, wobei das Kaliumsalz von Raltegravir in einer Menge von 300 mg pro Dosierungseinheit, auf Basis von freiem Phenol, vorliegt.

14. Gepresste Tablette nach Anspruch 1, umfassend:
| **Komponenten** | **Menge [mg]** |
|---|---|
| | **Intragranular** |
| Raltegravir-Kaliumsalz | 1303,20 |
| Crospovidon | 115,84 |
| Hypromellose | 28,96 |
| | **Extragranular** |
|---|---|
| Croscarmellose-Natrium | 128,00 |
| Magnesiumstearat | 24,00 |
| **Gewicht des Tablettenkerns** | **1600,00** |

15. Gepresste Tablette nach Anspruch 1, umfassend:
| **Komponenten** | **Menge [mg]** |
|---|---|
| | **Intragranular** |
| Raltegravir-Kaliumsalz | 651,60 |
| Crospovidon | 57,92 |
| Hypromellose | 14,48 |
| | **Extragranular** |
|---|---|
| Croscarmellose-Natrium | 64,00 |
| Magnesiumstearat | 12,00 |
| **Gewicht des Tablettenkerns** | **800,00** |

16. Gepresste Tablette nach Anspruch 1, umfassend:
| **Komponenten** | **Menge [mg]** |
|---|---|
| | **Intragranular** |
| Raltegravir-Kaliumsalz | 325,80 |
| Crospovidon | 28,96 |
| Hypromellose | 7,24 |
| | **Extragranular** |
|---|---|
| Croscarmellose-Natrium | 32,00 |
| Magnesiumstearat | 6,00 |
| **Gewicht des Tablettenkerns** | **400,00** |

## Revendications

1. Comprimé compressé destiné à une administration par voie orale, qui comprend :
(A) un composant intragranulaire comprenant :
(i) le sel de potassium de raltégravir, qui est présent sur une base de phénols libres en une quantité d'au moins environ 70 % en poids ;
(ii) un super-désintégrant inerte, qui est présent en une quantité située dans une plage allant de 5 % en poids à environ 10 % en poids ; et
(iii) un liant, qui est présent en une quantité située dans une plage allant d'environ 0,5 % en poids à environ 5 % en poids ;
et
(B) un composant extragranulaire comprenant :
(i) un super-désintégrant, qui est présent en une quantité située dans une plage allant d'environ 7 % en poids à environ 11 % en poids ; et
(ii) un lubrifiant, qui est présent en une quantité située dans une plage allant d'environ 0,5 % en poids à environ 2,5 % en poids ;
dans lequel le pourcentage en poids de chaque composant dans le comprimé compressé est basé sur le poids total du comprimé compressé et dans lequel le comprimé est exempt de charge.

2. Comprimé compressé selon la revendication 1, dans lequel le super-désintégrant inerte est choisi parmi le groupe consistant en crospovidone, croscarmellose sodique neutralisée et glycolate d'amidon sodique neutralisé.

3. Comprimé compressé selon la revendication 2, dans lequel le super-désintégrant inerte est la crospovidone.

4. Comprimé compressé selon la revendication 1, dans lequel le liant est choisi parmi le groupe consistant en hypromellose et hydroxypropylcellulose.

5. Comprimé compressé selon la revendication 4, dans lequel le liant est l'hypromellose.

6. Comprimé compressé selon la revendication 1, dans lequel le super-désintégrant est choisi parmi le groupe consistant en croscarmellose sodique, amidon et glycolate d'amidon sodique.

7. Comprimé compressé selon la revendication 6, dans lequel le super-désintégrant est la croscarmellose sodique.

8. Comprimé compressé selon la revendication 1, dans lequel le lubrifiant est choisi parmi le groupe consistant en stéarate de magnésium, acide stéarique, fumarate de stéaryle sodique et des mélanges de ceux-ci.

9. Comprimé compressé selon la revendication 8, dans lequel le lubrifiant est le stéarate de magnésium.

10. Comprimé compressé selon la revendication 1, dans lequel :
(A)(i)le sel de potassium de raltégravir est présent sur une base de phénols libres en une quantité d'au moins environ 75 % en poids ;
(A)(ii) le super-désintégrant inerte est présent en une quantité située dans une plage allant de 7 % en poids à environ 9 % en poids ;
(A)(iii) un liant est présent en une quantité située dans une plage allant d'environ 1,0 % en poids à environ 3,0 % en poids ;
(B)(i)le super-désintégrant est présent en une quantité située dans une plage allant d'environ 7 % en poids à environ 11 % en poids ; et
(B)(ii) le lubrifiant est présent en une quantité située dans une plage allant d'environ 1,0 % en poids à environ 2,0 % en poids ;
dans lequel le pourcentage en poids de chaque composant dans le comprimé compressé est basé sur le poids total du comprimé compressé.

11. Comprimé compressé selon la revendication 1, dans lequel le sel de potassium de raltégravir est présent sur une base de phénols libres en une quantité de 1 200 mg par dose unitaire.

12. Comprimé compressé selon la revendication 1, dans lequel le sel de potassium de raltégravir est présent sur une base de phénols libres en une quantité de 600 mg par dose unitaire.

13. Comprimé compressé selon la revendication 1, dans lequel le sel de potassium de raltégravir est présent sur une base de phénols libres en une quantité de 300 mg par dose unitaire.

14. Comprimé compressé selon la revendication 1, comprenant :
| **Composants** | **Quantité [mg]** |
|---|---|
| | **Intragranulaire** |
| Sel de potassium de | 1 303,20 |
| Crospovidone | 115,84 |
| Hypromellose | 28,96 |
| | **Extragranulaire** |
|---|---|
| Croscarmellose sodique | 128,00 |
| Stéarate de magnésium | 24,00 |
| **Poids du noyau du** | **1 600,00** |

15. Comprimé compressé selon la revendication 1, comprenant :
| **Composants** | **Quantité [mg]** |
|---|---|
| | **Intragranulaire** |
| Sel de potassium de | 651,60 |
| Crospovidone | 57,92 |
| Hypromellose | 14,48 |
| | **Extragranulaire** |
|---|---|
| Croscarmellose sodique | 64,00 |
| Stéarate de magnésium | 12,00 |
| **Poids du noyau du** | **800,00** |

16. Comprimé compressé selon la revendication 1, comprenant :
| **Composants** | **Quantité [mg]** |
|---|---|
| | **Intragranulaire** |
| Sel de potassium de | 325,80 |
| Crospovidone | 28,96 |
| Hypromellose | 7,24 |
| | **Extragranulaire** |
|---|---|
| Croscarmellose sodique | 32,00 |
| Stéarate de magnésium | 6,00 |
| **Poids du noyau du** | **400,00** |
